(19) Europäisches Patentamt
European Patent Office
Office européen des brevets

(11) Veröffentlichungsnummer: **0 040 176**
**B1**

(12) # EUROPÄISCHE PATENTSCHRIFT

(45) Veröffentlichungstag der Patentschrift: **15.02.84**

(51) Int. Cl.³: **C 07 D 213/61**

(21) Anmeldenummer: **81810175.0**

(22) Anmeldetag: **06.05.81**

(54) **Verfahren zur Herstellung von 2,3,5-Trichlorpyridin.**

(30) Priorität: **12.05.80 US 148849**

(43) Veröffentlichungstag der Anmeldung:
**18.11.81 Patentblatt 81/46**

(45) Bekanntmachung des Hinweises auf die Patenterteilung:
**15.02.84 Patentblatt 84/7**

(84) Benannte Vertragsstaaten:
**AT BE CH DE FR GB IT LI NL SE**

(56) Entgegenhaltungen:
**DE - A - 2 141 632**
**DE - A - 2 809 334**
**DE - A - 2 836 077**

(73) Patentinhaber: **CIBA-GEIGY AG, Patentabteilung Postfach, CH-4002 Basel (CH)**

(72) Erfinder: **Fäh, Hansjakob, Dr., Schlossweg, CH-4466 Ormalingen (CH)**
Erfinder: **Grieder, Alfred, Dr., Ob den Reben 15, CH-4461 Böckten (CH)**

**EP 0 040 176 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 2,3,5-Trichlorpyridin

Die vorliegende Erfindung betrifft ein Verfahren zur Herstellung von 2,3,5-Trichlorpyridin.

2,3,5-Trichlorpyridin ist ein wertvolles Zwischenprodukt zur Herstellung von herbizid wirksamen α-[4-(3',5'-Dichlorpyrid-2-yloxy)-phenoxy]-alkancarbonsäuren und deren Derivaten. Die Herstellung und Verwendung solcher α-[4-(3',5-Dichlorpyrid-2-yloxy)-phenoxy]-alkancarbonsäuren und ihrer Derivate ist beispielsweise in der US-Patenschrift Nr. 4092151 beschrieben.

Es ist bekannt, 2,3,5-Trichlorpyridin durch Umsetzung von N-Methyl-3,5-dichlor-2-pyridon mit Phosgen in Toluol als Lösungsmittel herzustellen. Das Verfahren wird in der Weise durchgeführt, dass man N-Methyl-3,5-dichlor-2-pyridon in eine toluolische Lösung von Phosgen einträgt und die Mischung in einem geschlossenen System 3 h auf 150° C erhitzt. Auf diese Weise wird 2,3,5-Trichlorpyridin in einer Ausbeute von etwa 90% der Theorie erhalten [„Ann. Chem." *486*, 78-79 (1931)]. Das zur Durchführung dieses Verfahrens als Ausgangsmaterial benötigte N-Methyl-3,5-dichlor-2-pyridon ist durch Umsetzung von 2-Aminopyridin mit Kaliumnitrit in schwefelsaurer Lösung zu 2-Pyridon [„Arch. Pharm." *240*, 350 (1902)], dessen Chlorierung zu 3,5-Dichlor-2-pyridon [„J. Org. Chem." *23*, 1614 (1958)] und anschliessende Methylierung desselben [„Ann. Chem." *486*, 74 (1931)] zugänglich.

Das bekannte Verfahren ist insofern nachteilig, als man das durch Chlorierung von 2-Pyridon erhaltene 3,5-Dichlor-2-pyridon erst in N-Methyl-3,5-dichlor-2-pyridon überführen muss, aus welchem dann bei der nachfolgenden Umsetzung mit Phosgen zu 2,3,5-Trichlorpyridin die N-Methylgruppe wieder abgespalten wird. Bei diesem Verfahren wird also intermediär eine Gruppe eingeführt, die im Endprodukt gar nicht vorhanden ist. Dies ist im Hinblick auf die Notwendigkeit der Durchführung einer zusätzlichen Verfahrensstufe und den damit verbundenen Aufwand sowie die durch die zusätzliche Verfahrensstufe verursachten Ausbeuteverluste und den Verbrauch an Chemikalien, die nicht zum strukturellen Aufbau des Endproduktes beitragen, unwirtschaftlich. Ferner muss wegen der hohen Reaktionstemperatur, bei der das bekannte Verfahren durchgeführt wird, in einem geschlossenen System unter Druck gearbeitet werden, wodurch sich der im Zusammenhang mit der grosstechnischen Durchführung des Verfahrens notwendige apparative Aufwand beträchtlich erhöht.

In den deutschen Offenlegungsschriften Nrn. 2836077, 2809334 und 2141632 sind verschiedene Verfahren zur Herstellung von chlorierten Pyridinen beschrieben. Diese Verfahren liefern durch Gasphasenchlorierung bei hohen Temperaturen entweder Produktgemische, die kein 2,3,5-Trichlorpyridin enthalten, oder sie erfordern den Einsatz von speziell vorchlorierten Zwischenprodukten, oder sie liefern durch Zinkstaubreduktion von Pentachlorpyridin oder Tetrachlorpyridin das gewünschte Produkt. Diese Verfahren erfordern also entweder hohe Reaktionstemperaturen oder erfordern zunächst die Synthese von hochchlorierten Produkten, die dann in zusätzlichen Reaktionsschritten wieder eliminert werden müssen. Insofern sind auch diese Verfahren unwirtschaftlich.

Es ist daher das Ziel der vorliegenden Erfindung, die Nachteile der bekannten Arbeitsweisen zu vermeiden, und ein Verfahren vorzusehen, das die Herstellung von 2,3,5-Trichlorpyridin in technischem Massstab auf einfache und wirtschaftliche Weise ermöglicht.

Gemäss vorliegender Erfindung wird vorgeschlagen, 2,3,5-Trichlorpyridin in der Weise herzustellen, dass man 3,5-Dichlor-2-pyridon bei 30-150° C in Gegenwart eines N,N-disubstituierten Formamids der Formel

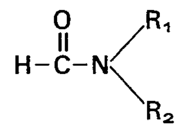

(I)

in welcher:

$R_1$ und $R_2$ gleich oder verschieden sein können und je eine Alkylgruppe mit 1-4 Kohlenstoffatomen bedeuten oder $R_1$ und $R_2$ zusammen mit dem Stickstoffatom die Pyrrolidino-, Piperidino- oder Morpholinogruppe bilden, und in Gegenwart eines inerten Lösungsmittels mit Phosgen umsetzt.

Die Umsetzung des 3,5-Dichlor-2-pyridons wird innerhalb des oben angegebenen Temperaturbereichs von 30-150° C vorzugsweise bei 50-90° C durchgeführt. Die Umsetzung erfolgt in der Regel bei Normaldruck. Lediglich bei Verwendung eines relativ niedrig siedenden Lösungsmittels und Anwendung einer hohen Reaktionstemperatur, beispielsweise einer Temperatur im Bereich von 100-150° C, kann es erforderlich sein, in einem geschlossenen System unter Druck zu arbeiten.

Als Lösungsmittel, in denen das erfindungsgemässe Verfahren durchgeführt werden kann, kommen allgemein solche in Betracht, die unter den Reaktionsbedingungen gegenüber den Reaktionsteilnehmern inert sind. Geeignete Lösungsmittel sind insbesondere aromatische Kohlenwasserstoffe, wie Benzol, Toluol oder Xylol, aliphatische Kohlenwasserstoffe, wie Hexan, Heptan oder Petroläther, cycloaliphatische Kohlenwasserstoffe, wie Cyclopentan und Cyclohexan, halogenierte aromatische Kohlenwasserstoffe, wie Chlorbenzol oder o-Dichlorbenzol, halogenierte aliphatische Kohlenwasserstoffe, wie Methylenchlorid, Chloroform, Kohlenstofftetrachlorid, 1,2-Dichloräthan und Trichloräthylen, sowie aliphatische Carbonsäureester, wie Äthylacetat und Isopropylacetat. Ein bevorzugtes Lösungsmittel ist Toluol.

Geeignete N,N-disubstituierte Formamide sind N,N-Dimethylformamid, N,N-Diäthylformamid, N,N-Dipropylformamid, N,N-Dibutylformamid,

N-Methyl-N-butylformamid sowie N-Formylpyrrolidin, N-Formylpiperidin und N-Formylmorpholin. Ein bevorzugtes N,N-disubstituiertes Formamid ist N,N-Dimethylformamid. Die N,N-disubstituierten Formamide werden in Mengen von 0,01-1,0 Mol pro Mol 3,5-Dichlor-2-pyridon, vorzugsweise 0,05 bis 0,15 Mol pro Mol 3,5-Dichlor-2-pyridon eingesetzt.

Das Phosgen wird erfindungsgemäss in der Regel in mindestens äquimolarer Menge oder in einem Überschuss eingesetzt. Zweckmässig verwendet man 1,0 bis 1,5 Mol Phosgen pro Mol 3,5-Dichlor-2-pyridon. Vorzugsweise setzt man 1,0 bis 1,3 Mol Phosgen pro Mol 3,5-Dichlor-2-pyridon ein. Nach beendigter Umsetzung das überschüssige Phosgen durch Zugabe einer wässerigen Lauge, wie wässerige Natronlauge oder wässerigen Ammoniak, zerstört.

Die Umsetzung des 3,5-Dichlor-2-pyridons mit Phosgen nimmt in der Regel 1-5 h in Anspruch und ist in den meisten Fällen in 2-4 h beendet.

Gemäss einer bevorzugten Ausführungsform des erfindungsgemässen Verfahrens suspendiert man das 3,5-Dichlor-2-pyridon in Toluol, trennt vorhandenes Wasser durch azeotrope Destillation ab, gibt 0,05 bis 0,15 Mol N,N-Dimethylformamid pro Mol 3,5-Dichlor-2-pyridon zu und leitet bei 75-80° C 1,0 bis 1,3 Mol Phosgen pro Mol 3,5-Dichlor-2-pyridon ein. Nach beendigter Phosgenzugabe lässt man 1 h bei 75-80° C nachreagieren und zerstört anschliessend das überschüssige Phosgen durch Zugabe von wässerigem Ammoniak. Danach trennt man die Phasen, destilliert das Lösungsmittel aus der organischen Phase ab und gewinnt das 2,3,5-Trichlorpyridin als Schmelze.

Mit dem erfindungsgemässen Verfahren wird es möglich, 2,3,5-Trichlorpyridin auf einfache und wirtschaftliche Weise in technischem Massstabe herzustellen. Das Verfahren vermeidet die nach dem bekannten Verfahren erforderliche N-Methylierung des 3,5-Dichlor-2-pyridons und kann unter wesentlich milderen Bedingungen durchgeführt werden. Damit wird die Gesamtausbeute gesteigert und der zur Durchführung des Verfahrens notwendige apparative Aufwand verringert.

Das erfindungsgemässe Verfahren wird durch das nachfolgende Beispiel näher erläutert:

*Beispiel:*

In einem 500-l-Emmailkessel werden 82,0 kg 3,5-Dichlor-2-pyridon, 330 kg Toluol und 3,8 kg N,N-Dimethylformamid vorgelegt. Dann wird die Mischung unter Rühren auf 75-80° C aufgeheizt und bei dieser Temperatur während 2-3 h 60,0 kg Phosgen eingeleitet. Nach beendigter Zugabe des Phosgens wird 1 h bei 75-80° C nachgerührt. Danach wird das Reaktionsgemisch auf 20-25° C abgekühlt und mit 12,0 kg 25%igem wässerigen Ammoniak verrührt. Nach Abtrennung der wässerigen Phase wird das Toluol bei Normaldruck abdestilliert. Als Rückstand erhält man 92,0 kg 2,3,5-Trichlorpyridin (95%ig; 96% der Theorie) als Schmelze, die beim Abkühlen erstarrt. Das Produkt schmilzt bei 47-48° C.

## Patentansprüche

1. Verfahren zur Herstellung von 2,3,5-Trichlorpyridin, dadurch gekennzeichnet, dass man 3,5-Dichlor-2-pyridon bei 30-150° C in Gegenwart von 0,01 bis 1,0 Mol pro Mol 3,5-Dichlor-2-pyridon eines N,N-disubstituierten Formamids der Formel

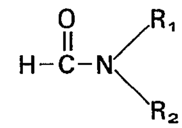

in welcher:

$R_1$ und $R_2$ gleich oder verschieden sein können und je eine Alkylgruppe mit 1-4 Kohlenstoffatomen bedeuten oder $R_1$ und $R_2$ zusammen mit dem Stickstoffatom die Pyrrolidino-, Piperidino- oder Morpholino-Gruppe bilden, und in Gegenwart eines inerten Lösungsmittels mit Phosgen umsetzt.

2. Verfahren nach Anspruch 1, dadurch gekennzeichnet, dass man die Umsetzung des 3,5-Dichlor-2-pyridons mit Phosgen bei einer Temperatur von 50-90° C durchführt.

3. Verfahren nach Anspruch 1, dadurch gekennzeichnet, dass man als inertes Lösungsmittel einen aromatischen Kohlenwasserstoff, einen aliphatischen Kohlenwasserstoff, einen cycloaliphatischen Kohlenwasserstoff, einen halogenierten aromatischen Kohlenwasserstoff, einen halogenierten aliphatischen Kohlenwasserstoff oder einen aliphatischen Carbonsäureester verwendet.

4. Verfahren nach Anspruch 1, dadurch gekennzeichnet, dass man als inertes Lösungsmittel Toluol verwendet.

5. Verfahren nach Anspruch 1, dadurch gekennzeichnet, dass man als N,N-disubstituiertes Formamid N,N-Dimethylformamid, N,N-Diäthylformamid, N,N-Dipropylformamid, N,N-Dibutylformamid, N-Methyl-N-butylformamid, N-Formylpyrrolidin, N-Formylpiperidin oder N-Formylmorpholin verwendet.

6. Verfahren nach Anspruch 1, dadurch gekennzeichnet, dass man als N,N-disubstituiertes Formamid N,N-Dimethylformamid verwendet.

7. Verfahren nach Anspruch 1, dadurch gekennzeichnet, dass man das N,N-disubstituierte Formamid in einer Menge von 0,05 bis 0,15 Mol pro Mol 3,5-Dichlor-2-pyridon verwendet.

8. Verfahren nach Anspruch 1, dadurch gekennzeichnet, dass man 1,0 bis 1,5 Mol Phosgen pro Mol 3,5-Dichlor-2-pyridon verwendet.

9. Verfahren nach Anspruch 1, dadurch gekennzeichnet, dass man 1,0 bis 1,3 Mol Phosgen pro Mol 3,5-Dichlor-2-pyridon verwendet.

10. Verfahren nach Anspruch 1, dadurch gekennzeichnet, dass man das 3,5-Dichlor-2-pyridon in Toluol suspendiert, 0,05 bis 0,15 Mol N,N-Dimethylformamid pro Mol 3,5-Dichlor-2-pyridon zugibt, bei 75-80° C 1,0 bis 1,3 Mol Phosgen pro Mol 3,5-Dichlor-2-pyridon einleitet, 1 h nachrührt, das überschüssige Phosgen durch Zugabe von wässerigem Ammoniak zerstört, die Phasen trennt und aus der organischen Phase das gebildete 2,3,5-Trichlorpyridin nach dem Abdestillieren des Lösungsmittels als Schmelze gewinnt.

## Claims

1. A process for producing 2,3,5-trichlorpyridine, which process comprises reacting 3,5-dichloro-2-pyridone at 30-150° C with phosgene in the presence of 0.01-1.0 mol, per mol of 3,5-dichloro-2-pyridone, of an N,N-disubstituted formamide of the formula

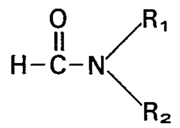

in which:

$R_1$ and $R_2$ can be identical or different and are each an alkyl group having 1-4 carbon atoms, or $R_1$ and $R_2$ together with the nitrogen atom form the pyrrolidino, piperidino or morpholino group, and in the presence of an inert solvent.

2. A process according to claim 1, wherein the reaction of 3,5-dichloro-2-pyridone with phosgene is performed at a temperature of 50-90° C.

3. A process according to claim 1, wherein the inert solvent used is an aromatic hydrocarbon, an aliphatic hydrocarbon, a cycloaliphatic hydrocarbon, a halogenated aromatic hydrocarbon, a halogenated aliphatic hydrocarbon or an aliphatic carboxylic acid ester.

4. A process according to claim 1, wherein the solvent used is toluene.

5. A process according to claim 1, wherein the N,N-disubstituted formamide used is N,N-dimethylformamide, N,N-diethylformamide, N,N-dipropylformamide, N,N-dibutylformamide, N-methyl-N-butylformamide, N-formylpyrrolidine, N-formylpiperidine or N-formylmorpholine.

6. A process according to claim 1, wherein the N,N-disubstituted formamide used is N,N-dimethylformamide.

7. A process according to claim 1, wherein the N,N-disubstituted formamide is used in an amount of 0.05-0.15 mol per mol of 3,5-dichloro-2-pyridone.

8. A process according to claim 1, wherein 1.0-1.5 mol of phosgene are used per mol of 3,5-dichloro-2-pyridone.

9. A process according to claim 1, wherein 1.0-1.3 mols of phosgene are used per mol of 3,5-dichloro-2-pyridone.

10. A process according to claim 1, wherein the 3,5-dichloro-2-pyridone is suspended in toluene, 0.05-0.15 mol of N,N-dimethylformamide per mol of 3,5-dichloro-2-pyridone is added, 1.0-1.3 mol of phosgene per mol of 3,5-dichloro-2-pyridone are introduced at 75-80° C, the reaction mixture is stirred for a further 1 h, the excess phosgene is reacted with aqueous ammonia, the phases are separated, and from the organic phase is obtained, after removal of the solvent by distillation, the formed 2,3,5-trichloropyridine in the form of a melt.

## Revendications

1. Procédé de préparation de 2,3,5-trichloropyridine, caractérisé par le fait qu'on fait réagir sur le phosgène, et en présence d'un solvant inerte, la 3,5-dichloro-2-pyridone à 30-150° C en présence de 0,01-1,0 mol par mole de 3,5-dichloro-2-pyridone d'un formamide N,N-disubstitué de formule:

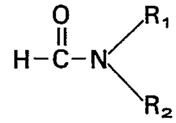

dans laquelle:

$R_1$ et $R_2$ peuvent être identiques ou différents et représentent chacun un groupe alkyle comportant 1-4 atomes de carbone, ou $R_1$ et $R_2$ constituent avec l'atome d'azote le groupe pyrrolidine, pipéridine ou morpholine.

2. Procédé suivant la revendication 1, caractérisé par le fait qu'on effectue la réaction de la 3,5-dichloro-2-pyridone avec le phosgène à une température de 50-90° C.

3. Procédé suivant la revendication 1, caractérisé par le fait qu'on utilise comme solvant inerte un hydrocarbure aromatique, un hydrocarbure aliphatique, un hydrocarbure cycloaliphatique, un hydrocarbure aromatique halogéné, un hydrocarbure aliphatique halogéné ou un ester aliphatique carboxylique.

4. Procédé suivant la revendication 1, caractérisé par le fait qu'on utilise le toluène comme solvant inerte.

5. Procédé suivant la revendication 1, caractérisé par le fait qu'on utilise comme formamide N,N-disubstitué le N,N-diméthylformamide, le N,N-diéthylformamide, le N,N-dipropylformamide, le N,N-dibutylformamide, le N-méthyl-N-butylformamide, la N-formylpyrrolidine, la N-formylpipéridine ou la N-formylmorpholine.

6. Procédé suivant la revendication 1, caractérisé par le fait qu'on utilise comme formamide N,N-disubstitué le N,N-diméthylformamide.

7. Procédé suivant la revendication 1, caractérisé par le fait qu'on utilise le formamide N,N-disubstitué à raison de 0,05-0,15 mol par mol de 3,5-dichloro-2-pyridone.

8. Procédé suivant la revendication 1, caractérisé par le fait qu'on utilise 1,0-1,5 mol de phosgène par mole de 3,5-dichloro-2-pyridone.

9. Procédé suivant la revendication 1, caractérisé par le fait qu'on utilise 1,0-1,3 mol de phosgène par mole de 3,5-dichloro-2-pyridone.

10. Procédé suivant la revendication 1, caractérisé par le fait qu'on met la 3,5-dichloro-2-pyridone en suspension dans le toluène, qu'on ajoute 0,05-0,15 mol de N,N-diméthylformamide par mole de 3,5-dichloro-2-pyridone, qu'on introduit à 75-80° C 1,0-1,3 mol de phosgène par mole de 3,5-dichloro-2-pyridone, qu'on agite ensuite pendant 1 h, qu'on détruit le phosgène en excès par addition d'ammoniaque aqueuse, qu'on sépare les phases et qu'on obtient la 2,3,5-trichloropyridine formée sous forme de masse fondue à partir de la phase organique, après distillation du solvant.